# EUROPÄISCHE PATENTSCHRIFT

(11) **EP 2 731 647 B1**
(45) Veröffentlichungstag und Bekanntmachung des Hinweises auf die Patenterteilung: **25.11.2015**
(21) Anmeldenummer: 12732795.5
(22) Anmeldetag: 03.07.2012
(51) Int. Cl.: A61M 5/28, A61M 5/31, B29C 45/44

(54) **MEHRKAMMER-SPRITZE**
MULTI-CHAMBER SYRINGE
SERINGUE MULTICHAMBRE

(30) Priorität: 15.07.2011 DE 102011107764
(43) Veröffentlichungstag der Anmeldung: 21.05.2014
(73) Patentinhaber: Sanner GmbH, 64625 Bensheim (DE)
(72) Erfinder: WEIDNER, Manfred, 64625 Bensheim (DE)
(74) Vertreter: Reiser & Partner
(86) Internationale Anmeldenummer: PCT/EP2012/002789
(87) Internationale Veröffentlichungsnummer: WO 2013/010631

(56) Entgegenhaltungen:
- EP-A1- 1 092 521
- EP-A2- 0 911 046
- DE-A1- 10 356 335
- DE-A1-102007 014 281
- DE-B3- 10 302 248
- GB-A- 705 392
- JP-A- 2007 260 349

## Beschreibung

Die Erfindung betrifft eine Mehrkammer-Spritze mit einem Spritzenkörper, dessen Wandung einen Hohlraum umschließt, wobei der Spritzenkörper einen Spritzenauslass und eine Öffnung zur Aufnahme eines in dem Hohlraum verschiebbaren Kolbens aufweist, wobei in dem Hohlraum zumindest ein verschiebbarer Stopfen angeordnet ist, der den Hohlraum in eine erste Kammer zur Aufnahme eines ersten Stoffes und in eine zweite Kammer zur Aufnahme eines zweiten Stoffes unterteilt, wobei der Spritzenkörper einen Kanal aufweist, durch den der erste Stoff aus der ersten Kammer in die zweite Kammer umströmen kann, wenn der Stopfen derart positioniert ist, dass der Kanal die erste Kammer mit der zweiten Kammer verbindet.

Eine derartige Mehrkammer-Spritze ist aus der DE 10 2007 014 281 A1 bekannt. Mehrkammer-Spritzen werden in der Medizin zur Verabreichung von Präparaten verwendet, die aus mehreren Stoffen bestehen. Die Stoffe können dabei sowohl in flüssiger als auch in fester Form vorliegen. In einer als Doppelkammerspritze ausgebildeten Mehrkammer-Spritze befinden sich in dem Spritzenkörper vor der Verabreichung getrennt voneinander zwei Stoffe, wobei beide Stoffe durch einen in dem Spritzenkörper angeordneten Stopfen getrennt sind. Das zweite Ende des Spritzenkörpers ist durch einen Kolben verschlissen. Der Stopfen verhindert ein unabsichtliches, vorheriges Mischen beider Stoffe. Dies ist insbesondere dann vorteilhaft, wenn die Stoffe untereinander unverträglich sind oder sich die Haltbarkeit der gemischten Stoffe reduziert.

Das Mischen der Stoffe erfolgt unmittelbar vor der Verabreichung. Dazu wird der Kolben in den Spritzenkörper eingedrückt, wodurch sich der Stopfen aufgrund des zwischen Kolben und Stopfen befindlichen inkompressiblen Mediums in Richtung auf die Spitze bewegt. Der Spritzenkörper weist in etwa mittig einen Bypass auf, der aus einer Ausbuchtung der Wandung des Spritzenkörpers gebildet ist. Erreicht der Stopfen den Bypass strömt der zwischen Stopfen und Kolben befindliche Stoff in den vor dem Stopfen befindlichen Hohlraum und vermischt sich mit dem dort befindlichen Stoff. Nach Abschluss des Mischvorgangs und nachdem die in dem Hohlraum befindliche Luft entfernt wurde, kann das mehrkomponentige Präparat verabreicht werden.

Bei dem aus der DE 10 2007 014 281 A1 bekannten Gegenstand ist nachteilig, dass die Herstellung des Spritzenkörpers mehrerer Schritte bedarf und daher aufwendig ist.

Der Erfindung liegt die Aufgabe zugrunde, eine Mehrkammer-Spritze zu entwickeln, welche insbesondere unter hygienischen Gesichtspunkten einfach und darüber hinaus auch kostengünstig herstellbar ist.

Diese Aufgabe wird durch die Merkmale der Ansprüche 1 und 11 gelöst. Auf vorteilhafte Ausgestaltungen nehmen die Unteransprüche Bezug.

GB 705392 und JP 2007260349 offenbaren Mehrkammer-Spritzen, die alle Merkmale des Oberbegriffs des Anspruch 1 beinhalten.

Zur Lösung der Aufgabe ist der Kanal durch eine auf der dem Hohlraum zugewandten Seite der Wandung ausgebildete Materialaussparung in der Wandung gebildet. Dabei kann die Materialaussparung so ausgebildet sein, dass der Außendurchmesser unverändert ist und kontinuierlich von dem Spritzenauslass zur Öffnung verläuft. Der Spritzenkörper weist dann hinsichtlich seines Außendurchmessers keinen Sprung auf, sondern verläuft durchgehend und kontinuierlich. Die Materialaussparung wird mittels Urformung hergestellt. Dabei bewirkt die Materialaussparung über den gesamten Bereich des Kanals eine Reduzierung der Wandstärke. Bei dieser Ausgestaltung ist vorteilhaft, dass der Spritzenkörper äußerlich von einer einfachen Einkammerspritze nicht zu unterscheiden ist, obwohl in der Innenwand ein Kanal ausgebildet ist, der als Bypass fungiert und zwei durch den Stopfen voneinander getrennte Kammern miteinander verbinden kann. Dies ermöglicht die Verwendung eines Spritzgießwerkzeuges, welches in Bezug auf die die Außenwand bildende Form identisch zu der Form eines für eine Einkammerspritze vorgesehenen Spritzgießwerkzeugs ist. Der Spritzenauslass geht in eine Hohlspitze über, welche vorzugsweise in Form eines Luer-Konus oder Luer-Lock ausgebildet ist. Diese Ausgestaltung ermöglicht die Befestigung handelsüblicher Kanülen auf dem Spritzenauslass.

Kolben und Stopfen können in verschiedenen Positionen in dem Hohlraum des Spritzenkörpers angeordnet sein. In einer ersten Position, der Lagerposition, befindet sich der Stopfen ausgehend von der Öffnung vor dem Kanal und der Spritzenauslass ist durch eine geeignete Dichtung, beispielsweise durch eine den Luer-konus dichtend umgebende Kappe verschlossen. In der Lagerposition ist der Kolben im Bereich der Öffnung in dem Hohlraum angeordnet, wobei Kolben und Stopfen jeweils dichtend an der Innenwand des Spritzenkörpers anliegen. Kolben und Stopfen begrenzen eine erste Kammer, während Stopfen und Spritzenauslass eine zweite Kammer begrenzen. In dieser Position ist der Kanal vollständig in der zweiten Kammer angeordnet.

Durch Druck auf den Kolben bewegt sich der Stopfen aufgrund des in der ersten Kammer befindlichen Stoffs in Richtung auf den Spritzenauslass bis der Stopfen in der Überströmposition zur Deckung mit dem Kanal gelangt. Sobald der Stopfen den Kanal überdeckt, verbindet der Kanal die erste Kammer mit der zweiten Kammer und der in der ersten Kammer befindliche Stoff strömt durch den Kanal in die zweite Kammer über. Der Stopfen verharrt in dieser Position bis der in der ersten Kammer befindliche Stoff vollständig in die zweite Kammer geströmt ist. Durch Druck auf den Kolben verkleinert sich das Volumen der ersten Kammer, bis der Kolben an dem Stopfen anliegt.

In dieser Position, der Gebrauchsposition, sind beide Medien vollständig miteinander vermischt, ggf. kann der Mischvorgang durch zusätzliches Schütteln der Mehrkammer-Spritze unterstützt werden. Durch weiteren Druck auf den Kolben verkleinert sich auch das Volumen der zweiten Kammer, so dass die gegebenenfalls in der zweiten Kammer befindliche Luft ausströmt. Anschließend können die miteinander vermischten Stoffe appliziert werden.

Der Kanal ist vorzugsweise als eine in Längsrichtung des Spritzenkörpers verlaufende Nut ausgebildet. Eine derartig ausgebildete Nut ist besonders einfach herstellbar. In diesem Zusammenhang ist denkbar, dass die Nut nach der Urformung des Spritzenkörpers in den Spritzenkörper eingebracht wird. Besonders bevorzugt wird die Nut jedoch bereits während der Urformung erzeugt, da sich bei dieser Herstellungsweise die Herstellungsschritte reduzieren. Die Nut ist so ausgebildet, dass deren Längserstreckung größer ist als die Längserstreckung, die Dicke, des Stopfens. Dadurch ist gewährleistet, dass der Kanal beide Kammern miteinander verbindet, wenn der Stopfen den Kanal überdeckt. Es ist auch denkbar, über den Innenumfang des Spritzenkörpers verteilt, mehrere Kanäle vorzusehen. Bei dieser Ausgestaltung ist aufgrund des vergrößerten Volumenstroms ein beschleunigter Überströmvorgang ermöglicht.

Der Kanal kann einen ersten Abschnitt aufweisen, in dem der Nutgrund der Nut schräg zur Mittelachse des Spritzenkörpers verläuft. Durch den schräg verlaufenden Abschnitt reduziert sich der Querschnitt des Kanals kontinuierlich, was insbesondere in dem Einströmbereich des Kanals zu einer Verbesserung der Strömungsverhältnisse während des überströmvorgangs führt. Des Weiteren ermöglicht der schräg verlaufende Abschnitt eine einfache Herstellbarkeit des Spritzenkörpers, insbesondere durch eine Verbesserung der Entformbarkeit des zum Spritzgießwerkzeug gehörenden Kerns.

Der Kanal kann einen zweiten Abschnitt aufweisen, in dem der Nutgrund parallel zur Mittelachse des Spritzenkörpers verläuft. Dieser Abschnitt ist vorzugsweise dem Bereich zugeordnet, in dem der Stopfen in der Überströmposition den Kanal überdeckt. In diesem Bereich ergibt sich ein gleichbleibender Querschnitt ohne Strömungshindernis.

Der erste Abschnitt ist vorzugsweise näher an der Öffnung und der zweite Abschnitt näher an dem Spritzenauslass gelegen. Hierbei ist vorteilhaft, dass sich die Strömungsverhältnisse während des Einströmens des Stoffs in den Kanal verbessern. Der zweite Abschnitt kann hingegen im Bereich der zweiten Kammer in eine Kante münden, so dass der überströmende Stoff umgelenkt wird und sich dadurch der Mischvorgang mit dem in der zweiten Kammer befindlichen Stoff verbessert wird.

Der Innendurchmesser des Spritzenkörpers erweitert sich ausgehend von dem Spritzenauslass in Richtung auf die Öffnung. Dabei ergibt sich eine wenigstens abschnittsweise konisch ausgebildete Innenwand des Spritzenkörpers. Diese Formgebung verbessert die Entformbarkeit des Kerns des Spritzgießwerkzeugs.

Zur Erweiterung des Innendurchmessers verläuft die Innenwand des Spritzenkörpers unter einem Öffnungswinkel zur Mittelachse. Dabei kann die Erweiterung des Innendurchmessers in Bereichen erfolgen, wobei insbesondere der Öffnungswinkel des dem Kanal zugeordneten ersten Bereichs größer sein kann als der dem Spritzenauslass zugeordneten zweite Bereich und/oder der der Öffnung zugeordneten dritte Bereich. Die durch die Vergrößerung des Innendurchmessers sich bildenden konischen Schrägen werden auch als Formschrägen bezeichnet. Diese verbessern die Entformbarkeit des zum Spritzgießwerkzeug gehörenden Kerns. Wird der Kanal in einem Zug mit der Urformung des Spritzenkörpers hergestellt, wird zur Formgebung des Kanals ein Werkzeug benötigt, welches vorzugsweise aus dem Kern ausgebildet ist. Dieses Werkzeug kann allerdings scharfkantig ausgebildet sein, was während des Entformungsvorgangs Kratzer in der Innenwand des Spritzenkörpers verursachen kann. Aufgrund der in Bereich des Kanals angeordneten größeren Formschräge, beziehungsweise unter einem größeren Winkel verlaufenden Querschnittserweiterung, wird der Kern samt Werkzeug zur Formung des Kanals während des Entformens bereits nach einer kurzen Wegstrecke freigestellt und weist keinen Kontakt zur Innenwand des Spritzenkörpers mehr auf. Eine Kratzerbildung an der Innenwand wird dadurch vermieden.

Des Weiteren steigt durch den sich in Richtung auf den Spritzenauslass verringernden Durchmesser der Anpressdruck an den in dem Spritzenkörper geführten Stopfen an, während dieser sich von der Lagerposition in die Überströmposition bewegt. Dadurch ist auch sicher gestellt, dass der Stopfen in der Oberströmposition verharrt während der Stoff überströmt.

Der Öffnung des Spritzenkörpers kann eine ringförmige Griffplatte zugeordnet sein. Die Griffplatte dient als Griff während des Verabreichens der Fluide und ermöglicht eine gezielte Steuerung des Kolbens. Aufgrund der ringförmigen Ausgestaltung ist der Spritzenkörper während einer maschinellen Befüllung der Mehrkammer-Spritze einfacher im Handling.

Der Spritzenkörper besteht vorzugsweise aus einem Kunststoff-Spritzguss-Werkstoff, insbesondere aus Cyclo-Olefin-Copolymer. Cyclo-Olefin-Copolymer ist ein amorphes und dadurch transparentes spritzgießfähiges Olefin. Ferner weist der Werkstoff neben hoher Steifigkeit und Härte eine gute Biokompabilität auf.

Stopfen und Kolben bestehen vorzugsweise aus einem elastomeren Werkstoff, insbesondere aus Bromobutyl-Kautschuk (BIIR). Bromobutyl ist ein durch Halogene modifizierter Isobuten-Isopren-Kautschuk aus der Gruppe der synthetischen Elastomere. Bromobutyl zeichnet sich durch eine gute Beständigkeit gegen Säuren und Basen und eine sehr geringe Gasdurchlässigkeit aus. Dadurch können Stoffe über einen längeren Zeitraum in der Mehrkammer-Spritze gelagert werden.

Stopfen und Kolben können mit umlaufenden Rippen versehen sein. Die umlaufenden Rippen verbessern die Dichtwirkung von Stopfen und Kolben ohne das Gleitverhalten zu beeinträchtigen. Zur Verbesserung des Gleitverhaltens und zur Vermeidung des Rutschgleitens (Stick-Slip) können Stopfen, Kolben und Innenwand des Spritzenkörpers mit einer Beschichtung, vorzugsweise auf Silikon-Basis versehen sein.

Vorzugsweise ist der Spritzenkörper einschließlich des in seiner Wandung ausgebildeten Kanals im Wege des Spritzgießverfahrens hergestellt. Hierbei ist vorteilhaft, dass der Spritzenkörper samt Kanal in einem Zug hergestellt wird, ohne dass zusätzliche nachgeschaltete Fertigungsschritte erforderlich sind. Dadurch vereinfacht sich die Fertigung der Mehrkammer-Spritze und die Herstellungskosten verringern sich.

Bei dem erfindungsgemäßen Verfahren zur Herstellung einer Mehrkammer-Spritze nach einem der vorherigen Ansprüche wird der Spritzenkörper mittels Kunststoff-Spritzguss geformt, wobei der Kanal während des Spritzguss-Vorgangs gleichzeitig mit der Urformung des Spritzenkörpers erzeugt wird. Durch ein geeignetes Werkzeug, welches mit dem die Innenwand des Spritzenkörpers bildenden Kern des Spritzgießwerkzeugs wirkverbunden ist, ist eine integrative Herstellung des Spritzenkörpers samt Kanal möglich.

Zur Urformung des Spritzenkörpers und gleichzeitigen Herstellung des Kanals kann aus dem die Innenwand des Spritzenkörpers bildenden Kern ein Schrägschieber hervorstehen und so auf der Innenwand des Spritzenkörpers den in die Wandung ragenden Kanal bildet, wobei der Schrägschieber nach Abschluss des Spritzgießvorganges in den Kern eingezogen wird und danach der Kern in Richtung der Öffnung aus dem Hohlraum des Spritzenkörpers herausgezogen wird. Der Schrägschieber ist insbesondere im Hinblick auf seine geringen Abmessungen vorteilhaft. Durch die geringen Abmessungen können in den Kern Kühleinrichtungen integriert werden, die bis in den Bereich des Spritzenauslasses ragen.

Einige Ausgestaltungen der erfindungsgemäßen Mehrkammer-Spritze werden nachfolgend anhand der Figuren näher erläutert. Diese zeigen, jeweils schematisch:
- Fig. 1: die Mehrkammer-Spritze im Schnitt in der Lagerposition;
- Fig. 2: die Mehrkammer-Spritze im Schnitt in der Umströmposition;
- Fig. 3: die Mehrkammer-Spritze im Schnitt in der Gebrauchsposition;
- Fig. 4: die Mehrkammer-Spritze im Schnitt entleert;
- Fig. 5: der Spritzenkörper in der Längsansicht;
- Fig. 6: der Spritzenkörper in räumlicher Darstellung;
- Fig. 7: der Spritzenkörper im Längsschnitt;
- Fig. 8: der Spritzenkörper im Längsschnitt;
- Fig. 9: im Detail der Kanal im Längsschnitt;
- Fig. 10: die Querschnittserweiterung der Innenwand schematisch;
- Fig. 11: den Spritzenkörper im Querschnitt;
- Fig. 12: das Spritzgießwerkzeug im Urformvorgang;
- Fig. 13: den Spritzenkörper teilweise aus dem Spritzgießwerkzeug entformt;
- Fig. 14: den Spritzenkörper vollständig aus dem Spritzgießwerkzeug entformt.

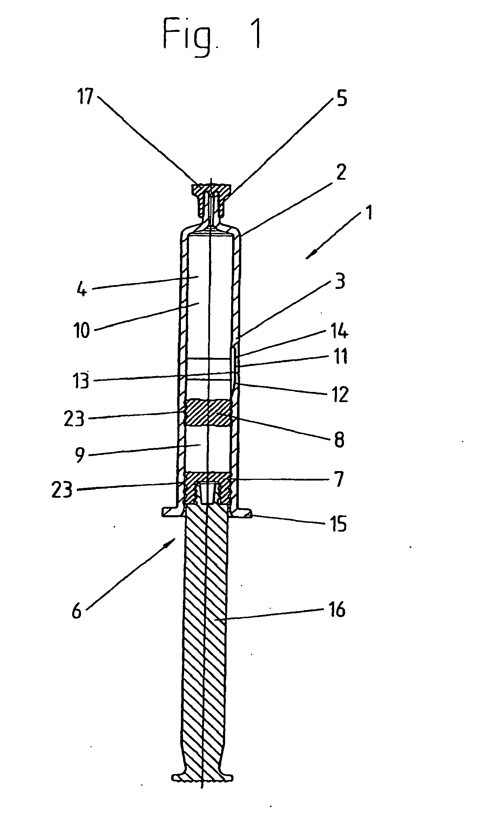

Figur 1 zeigt eine Mehrkammer-Spritze 1 mit einem Spritzenkörper 2, dessen Wandung 3 einen Hohlraum 4 umschließt. Der Spritzenkörper 2 ist als Spritzgussteil ausgebildet und besteht aus einem transparenten, durchsichtigen und spritzgießfähigen Kunststoff von ausreichender Härte. Ein derartiger Werkstoff ist beispielsweise Cyclo-Olefin-Copolymer (COC). Der Spritzenkörper 2 weist einen Spritzenauslass 5 auf, welcher die Form einer Spitze aufweist und sich in Richtung des freien Endes konisch verjüngt. Vorzugsweise weist der Spritzenauslass 5 die Form eines Luer-Konus auf. In einer weiteren Ausgestaltung kann der Spritzenauslass 5 auch ein Gewinde in Form eines Luer-Lock aufweisen. Der Spritzenauslass 5 ist mit einer Kappe 17 aus elastischem Kunststoff, vorzugsweise aus Bromobutyl (BIIR) verschlossen.

Der Spritzenkörper weist eine Öffnung 6 zur Aufnahme eines in dem Hohlraum 4 verschiebbaren Kolbens 7 auf, wobei in dem Hohlraum 4 zumindest ein verschiebbarer Stopfen 8 angeordnet ist, der den Hohlraum 4 in eine erste Kammer 9 zur Aufnahme eines ersten Stoffs und in eine zweite Kammer 10 zur Aufnahme eines zweiten Stoffs unterteilt. Die Stoffe können sowohl flüssig als auch pulverförmig vorliegen. Das Volumen der zweiten Kammer 10 ist so bemessen, dass die zweite Kammer 10 sowohl den Stoff der ersten Kammer 9 als auch den Stoff der zweiten Kammer 10 zuzüglich eines Zusatzvolumens zur Verbesserung des Mischvorgangs aufnehmen kann. Der Kolben 7 und der Stopfen 8 bestehen aus einem elastomeren Werkstoff, vorzugsweise aus Bromobutyl-Kautschuk (BIIR). Der Stopfen 8 und der Kolben 7 sind zur Verbesserung der Dichtwirkung mit umlaufenden Rippen 23 versehen.

Zur Verbindung von erster Kammer 9 und zweiter Kammer 10 weist der Spritzenkörper 2 einen Kanal 11 auf, durch den der erste Stoff aus der ersten Kammer 9 in die zweite Kammer 10 umströmen kann, wenn der Stopfen 8 derart positioniert ist, dass der Kanal 11 die erste Kammer 9 mit der zweiten Kammer 10 verbindet. Erfindungsgemäß ist der Kanal 11 durch eine auf der dem Hohlraum 4 zugewandten Seite der Wandung 3 ausgebildete Materialaussparung in der Wandung 3 gebildet. Der Kolben 7 ist mit einem Innengwinde versehen, in welches eine Kolbenstange 16 eingeschraubt ist.

Der Kolben 7 und der Stopfen 8 können in verschiedenen Positionen in dem Hohlraum 4 des Spritzenkörpers 2 angeordnet sein. In einer ersten Position, der Lagerposition, befindet sich der Stopfen 8 ausgehend von der Öffnung 6 vor dem Kanal 11 und der Spritzenauslass 5 ist durch eine Kappe 17 verschlossen. In der Lagerposition ist der Kolben 7 im Bereich der Öffnung 6 in dem Hohlraum 4 angeordnet, wobei Kolben 7 und Stopfen 8 jeweils dichtend an der Innenwand des Spritzenkörpers 2 anliegen. Kolben 7 und Stopfen 8 begrenzen die erste Kammer 9, während Stopfen 8 und Spritzenauslass 2 die zweite Kammer 10 begrenzen. In dieser Position ist der Kanal 11 vollständig in der zweiten Kammer 10 angeordnet.

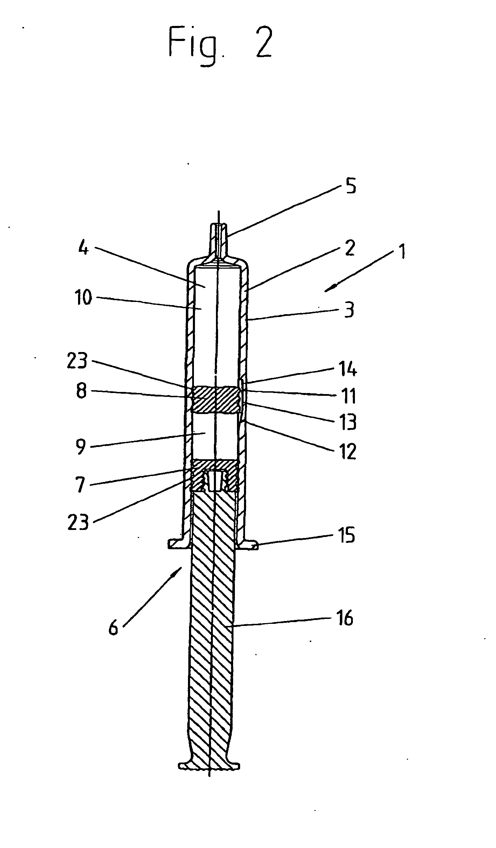

Nach Entfernen der Kappe 17 bewegt sich der Stopfen 8 durch Druck auf den Kolben 7 aufgrund des in der ersten Kammer 9 befindlichen meist inkompressiblen Stoffs in Richtung auf den Spritzenauslass 5 bis der Stopfen 8 in der Überströmposition zur Deckung mit dem Kanal 11 gelangt. Diese Position ist in Figur 2 gezeigt.

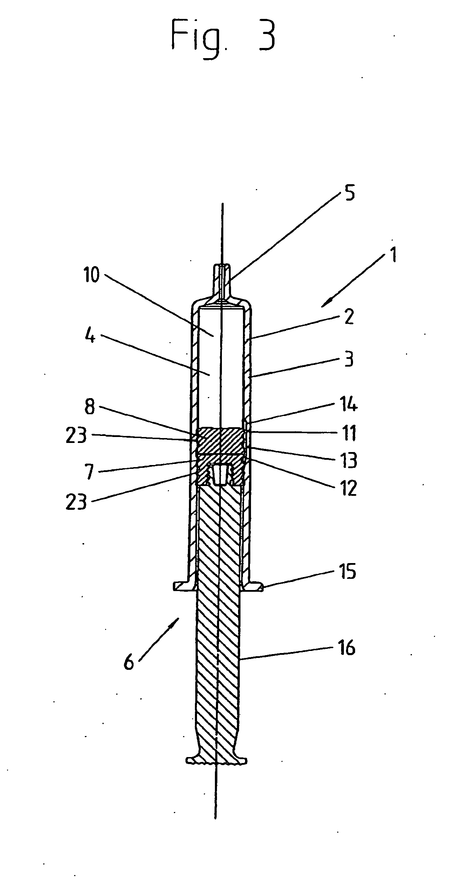

Sobald der Stopfen 8 den Kanal 11 überdeckt, verbindet der Kanal 11 die erste Kammer 9 mit der zweiten Kammer 10 und der in der ersten Kammer 9 befindliche Stoff strömt durch den Kanal 11 in die zweite Kammer 10 über. Der Stopfen 8 verharrt in dieser Position bis der in der ersten Kammer 9 befindliche Stoff vollständig in zweite Kammer geströmt 10 ist. Dazu ist der Anpressdruck des Stopfens 8 an die Innenwand des Spritzenkörpers 2 so ausgelegt, dass die Kraft die zum Bewegen des Stopfens 8 in dem Spritzenkörper 2 erforderlich ist, größer ist als der Widerstand der dem durch den Kanal 11 strömenden Stoff entgegengesetzt wird. Durch Druck auf den Kolben 7 verkleinert sich das Volumen der ersten Kammer 9, bis der Kolben 7 an dem Stopfen 8 anliegt. Diese Position ist in Figur 3 gezeigt.

In dieser Position, der Gebrauchsposition, werden beide Stoffe miteinander vermischt und durch weiteren Druck auf den Kolben 7 verkleinert sich auch das Volumen der zweiten Kammer 10, so dass die gegebenenfalls in der zweiten Kammer 10 befindliche Luft ausströmt. Anschließend können die miteinander vermischten Stoffe appliziert werden, nachdem eine Nadel auf den Spritzenauslass 5 aufgesetzt wurde.

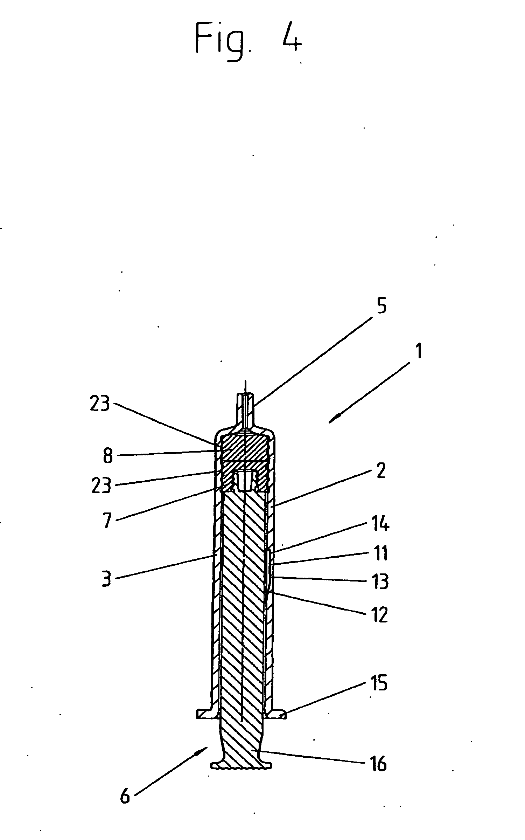

In Figur 4 ist die Position von Kolben 7 und Stopfen 8 nach dem Applizieren der beiden miteinander vermischten Stoffe gezeigt.

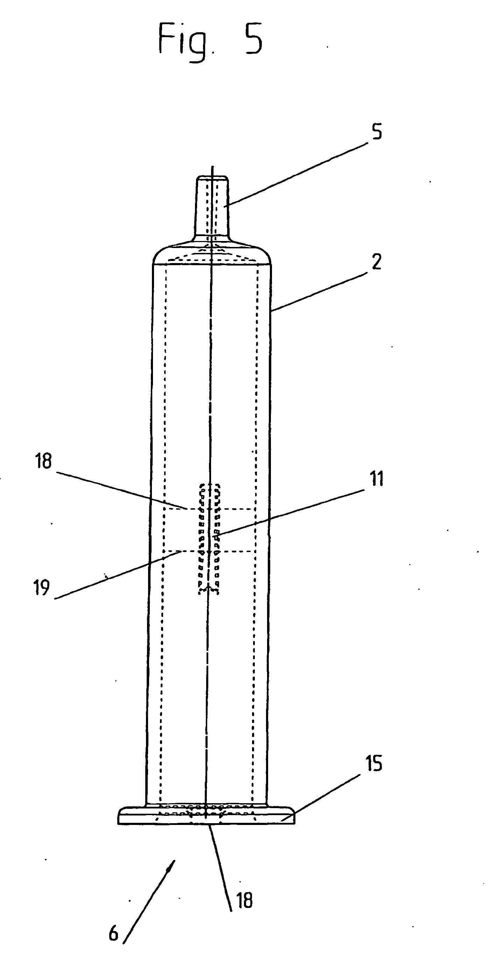

Figur 5 zeigt eine Längsansicht des Spritzenkörpers 2 der zuvor beschriebenen Mehrkammer-Spritze 1. In der Ansicht sind die in dem Hohlraum 4 des Spritzenkörpers 2 angeordneten Einrichtungen, beispielsweise der Kanal 11 gestichelt dargestellt.

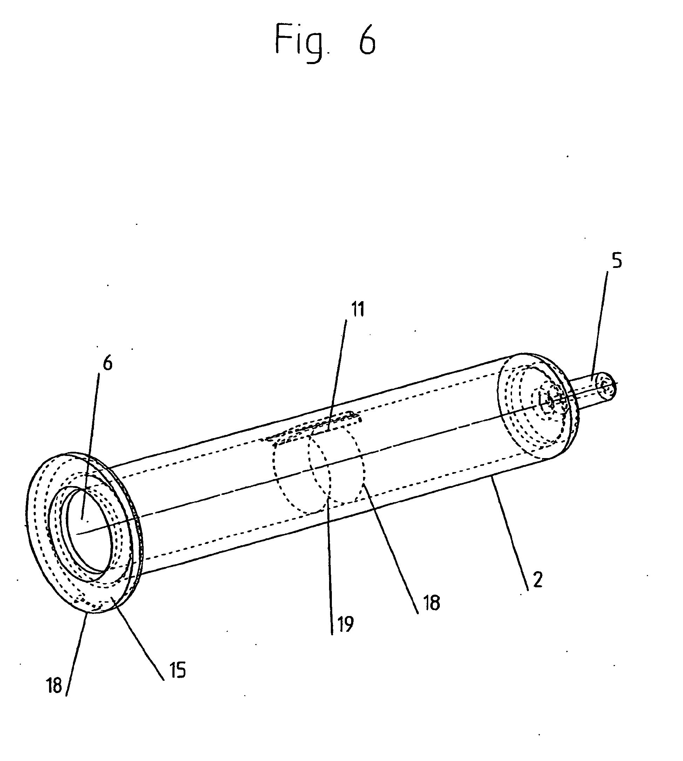

Figur 6 zeigt denselben Spritzenkörper in räumlicher Darstellung. Es ist zu erkennen, dass der Öffnung 6 des Spritzenkörpers 2 eine Griffplatte 15 zugeordnet ist, welche ringförmig ausgebildet ist.

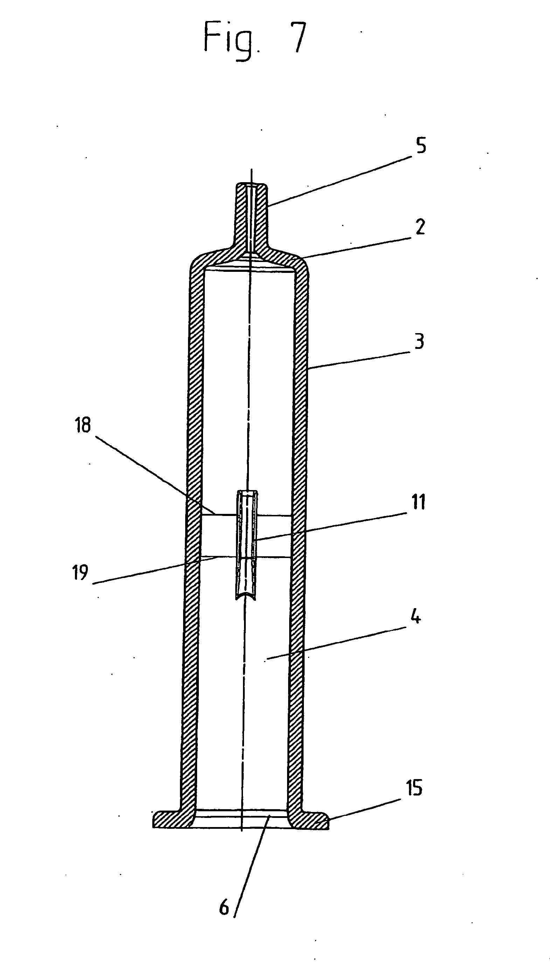

Figur 7 zeigt den zuvor beschriebenen Spritzenkörper 2 im Längsschnitt. Dabei ist in der Draufsicht der Kanal 11 dargestellt, welcher als eine in Längsrichtung des Spritzenkörpers 2 verlaufende Nut ausgebildet ist. Der Kanal 11 ist aus der Wandung 3 des Spritzenkörpers 2 ausgebildet, wodurch die Wandung 3 in diesem Bereich eine verringerte Dicke aufweist.

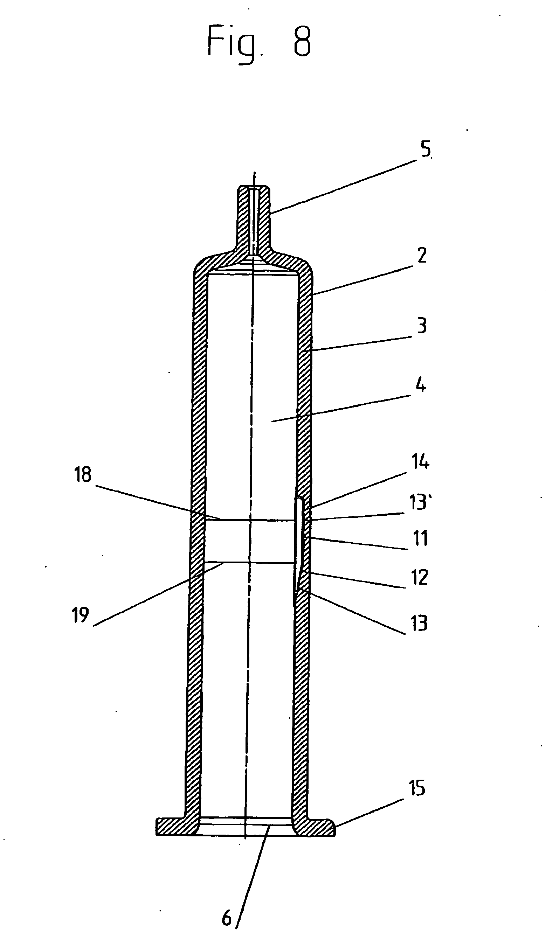

Figur 8 zeigt den zuvor beschriebenen Spritzenkörper ebenfalls im Längsschnitt, wobei der Kanal 11 in dieser Darstellung ebenfalls geschnitten dargestellt ist. Dadurch ist zu erkennen, dass der Kanal 11 einen ersten Abschnitt 12 aufweist, in dem der Nutgrund 13 der Nut schräg zur Mittelachse des Spritzenkörpers 2 verläuft und einen zweiten Abschnitt 14 aufweist, in dem der Nutgrund 13' parallel zur Mittelachse des Spritzenkörpers 2 verläuft. Dabei ist der erste Abschnitt 12 näher an der Öffnung 6 und der zweite Abschnitt 14 näher an dem Spritzenauslass 5 gelegen.

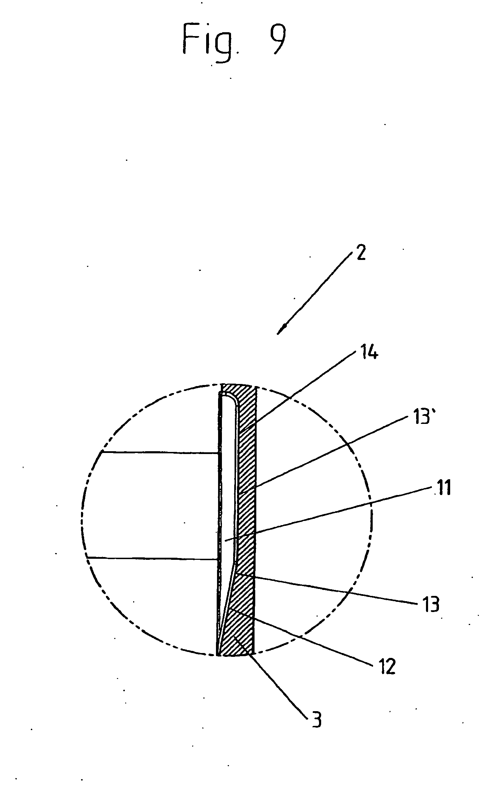

Figur 9 zeigt ausschnittsweise den in Figur 8 beschriebenen Kanal 11.

Der Innendurchmesser des Spritzenkörpers 2 erweitert sich ausgehend von dem Spritzeriauslass 5 in Richtung auf die Öffnung 6. Die Erweiterung des Innendurchmessers verläuft jedoch nicht kontinuierlich, sondern bereichsweise. Dabei verläuft die Innenwand des Spritzenkörpers 2 unter einem Öffnungswinkel zur Mittelachse. Die Erweiterung des Innendurchmessers erfolgt in Bereichen, wobei der Öffnungswinkel des dem Kanal 11 zugeordneten ersten Bereichs größer ist als der dem Spritzenauslass 5 zugeordnete zweite Bereich und größer ist als der der Öffnung 6 zugeordnete dritte Bereich. Der zweite Bereich beginnt bei dem Spritzenauslass und endet an der ersten Linie 18, welche im Bereich des Kanals 11 zu erkennen ist. Der erste Bereich, der dem Kanal zugeordnete Bereich ist durch die erste Linie 18 und die zweite Linie 19 begrenzt. Der dritte Bereich beginnt an der zweiten Linie 19 und endet an der Öffnung 6. Der Öffnungswinkel des zweiten Bereichs und des dritten Bereichs sind gleich und der Öffnungswinkel des ersten Bereichs ist größer als der Öffnungswinkel des zweiten Bereichs und des dritten Bereichs.

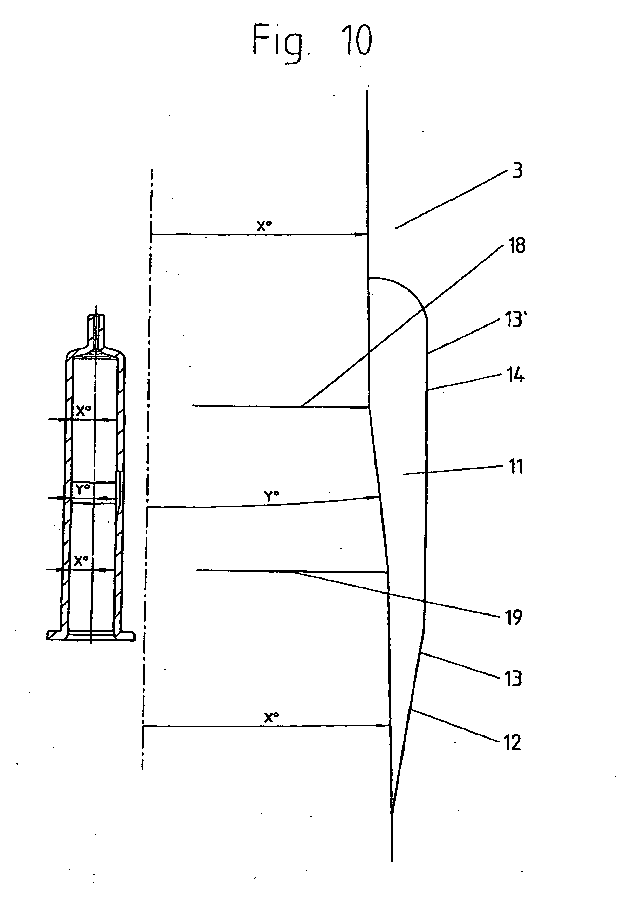

Diese Stufung der Öffnungswinkel ist in Figur 10 schematisch dargestellt.

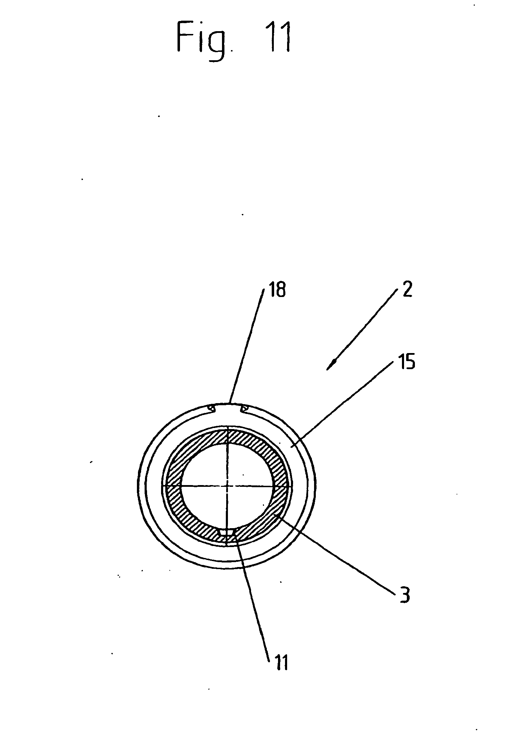

Figur 11 zeigt den zuvor beschriebenen Spritzenkörper 2 im Querschnitt. Im Bereich der Griffplatte 15 des Spritzenkörpers 2 ist ein Angussbereich 18 angeordnet. In diesem Bereich wird das Material des Spritzenkörpers 2 während des Spritzgießvorgangs zugeführt.

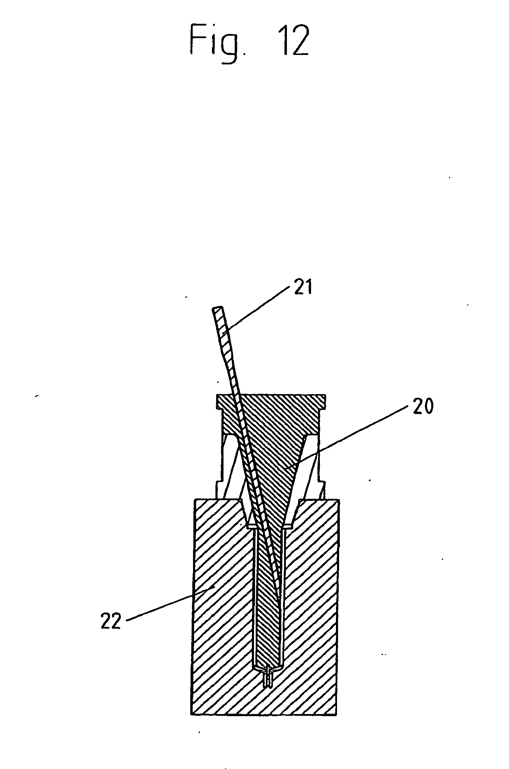

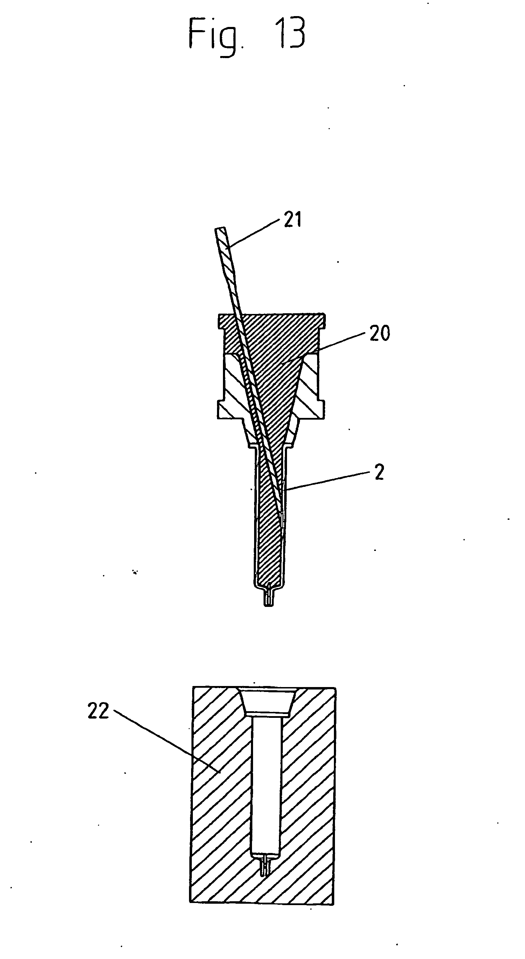

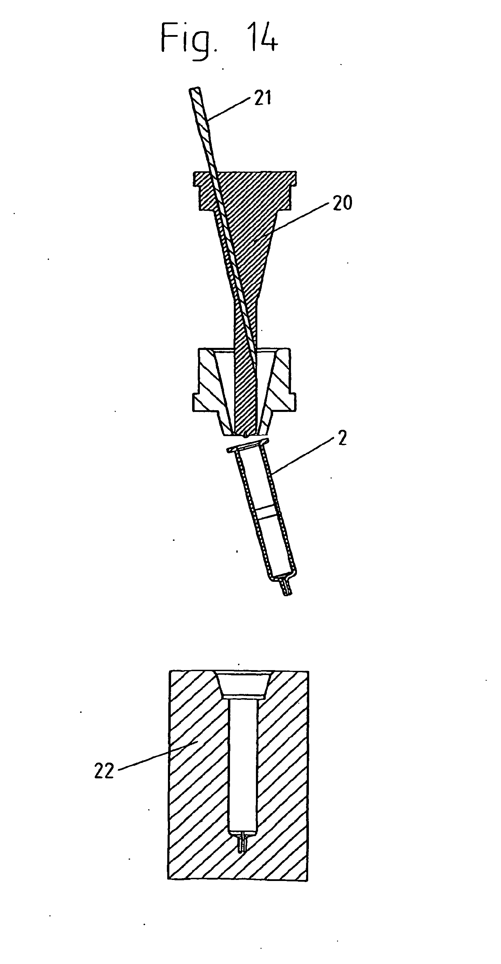

Die Figuren 12 bis 14 zeigen ein Herstellungsverfahren und ein Werkzeug zur Herstellung eines Spritzenkörpers 2 für eine Mehrkammer-Spritze 1 wie zuvor beschrieben. Der Spritzenkörper 2 wird im Spritzgießverfahren hergestellt, wobei der Kanal 11 während des Spritzguss-Vorgangs gleichzeitig mit der Urformung des Spritzenkörpers 2 erzeugt wird. Dazu wird vor dem Zuführen des Werkstoffs aus dem die Innenwand des Spritzenkörpers 2 bildenden Kern 20 ein Schrägschieber 21 herausgefahren, welcher so auf der beim Zuführen des Kunststoffs sich bildenden Innenwand des Spritzenkörpers 2 den in die Wandung 3 ragenden Kanal 11 bildet. Nach Abschluss des Spritzgießvorganges wird der Schrägschieber 21 in den Kern 20 eingezogen und der Kern 20 samt Spritzenkörper 2 von dem Spritzgießwerkzeug 22 entfernt, wie in Figur 14 zu erkennen ist. Anschließend wird der Kern 20 in Richtung der Öffnung 6 aus dem Hohlraum 4 des Spritzenkörpers 2 herausgezogen.

## Patentansprüche

1. Mehrkammer-Spritze (1) mit einem Spritzenkörper (2), dessen Wandung (3) einen Hohlraum (4) umschließt, wobei der Spritzenkörper (2) einen Spritzenauslass (5) und eine Öffnung (6) zur Aufnahme eines in dem Hohlraum (4) verschiebbaren Kolbens (7) aufweist, wobei in dem Hohlraum (4) zumindest ein verschiebbarer Stopfen (8) angeordnet ist, der den Hohlraum (4) in eine erste Kammer (9) zur Aufnahme eines ersten Stoffs und in eine zweite Kammer (10) zur Aufnahme eines zweiten Stoffs unterteilt, wobei der Spritzenkörper (2) einen Kanal (11) aufweist, durch den der erste Stoff aus der ersten Kammer (9) in die zweite Kammer (10) umströmen kann, wenn der Stopfen (8) derart positioniert ist, dass der Kanal (11) die erste Kammer (9) mit der zweiten Kammer (10) verbindet, wobei der Kanal (11) durch eine auf der dem Hohlraum (4) zugewandten Seite der Wandung (3) ausgebildete Materialaussparung in der Wandung (3) gebildet ist, **dadurch gekennzeichnet, dass** sich der Innendurchmesser des Spritzenkörpers (2) ausgehend von dem Spritzenauslass (5) in Richtung auf die Öffnung (6) erweitert und dass zur Erweiterung des Innendurchmessers die Innenwand des Spritzenkörpers (2) unter einem Öffnungswinkel zur Mittelachse verläuft, wobei die Erweiterung des Innendurchmessers in Bereichen erfolgt und wobei der Öffnungswinkel des dem Kanal (11) zugeordneten ersten Bereichs größer ist als der dem Spritzenauslass (5) zugeordnete zweite Bereich und der der Öffnung (6) zugeordnete dritte Bereich.

2. Mehrkammer-Spritze nach Anspruch 1, **dadurch gekennzeichnet, dass** der Kanal (11) als eine in Längsrichtung des Spritzenkörpers (2) verlaufende Nut ausgebildet ist.

3. Mehrkammer-Spritze nach Anspruch 2, **dadurch gekennzeichnet, dass** der Kanal (11) einen ersten Abschnitt (12) aufweist, in dem der Nutgrund (13) der Nut schräg zur Mittelachse des Spritzenkörpers (2) verläuft.

4. Mehrkammer-Spritze nach einem der Ansprüche 1 bis 3, **dadurch gekennzeichnet, dass** der Kanal (11) einen zweiten Abschnitt (14) aufweist, in dem der Nutgrund (13') parallel zur Mittelachse des Spritzenkörpers (2) verläuft.

5. Mehrkammer-Spritze nach Anspruch 4, **dadurch gekennzeichnet, dass** der erste Abschnitt (12) näher an der Öffnung (6) und dass der zweite Abschnitt (14) näher an dem Spritzenauslass (5) gelegen ist.

6. Mehrkammer-Spritze nach einem der Ansprüche 1 bis 5, **dadurch gekennzeichnet, dass** der Öffnung (6) des Spritzenkörpers (2) eine ringförmige Griffplatte (15) zugeordnet ist.

7. Mehrkammer-Spritze nach einem der Ansprüche 1 bis 6, **dadurch gekennzeichnet, dass** der Spritzenkörper (2) aus einem Kunststoff-Spritzguss-Werkstoff, vorzugsweise aus Cyclo-Olefin-Copolymer gebildet ist.

8. Mehrkammer-Spritze nach einem der Ansprüche 1 bis 7, **dadurch gekennzeichnet, dass** Stopfen (8) und Kolben (7) aus einem elastomeren Werkstoff, vorzugsweise aus Bromobutyl-Kautschuk gebildet sind.

9. Mehrkammer- Spritze nach einem der Ansprüche 1 bis 8, **dadurch gekennzeichnet, dass** Stopfen (8) und Kolben (7) mit umlaufenden Rippen (23) versehen sind.

10. Mehrkammer-Spritze nach einem der Ansprüche 1 bis 9, **dadurch gekennzeichnet, dass** der Spritzenkörper (2) einschließlich des in seiner Wandung ausgebildeten Kanals (11) im Wege des Spritzgießverfahrens hergestellt ist.

11. Verfahren zur Herstellung einer Mehrkammer-Spritze nach einem der vorherigen Ansprüche bei dem der Spritzenkörper (2) mittels Kunststoff-Spritzguss geformt wird, wobei der Kanal (11) während des Spritzguss-Vorgangs gleichzeitig mit der Urformung des Spritzenkörpers (2) erzeugt wird.

12. Verfahren nach Anspruch 11, **dadurch gekennzeichnet, dass** zur Urformung des Spritzenkörpers (2) und gleichzeitigen Herstellung des Kanals (11) aus dem die Innenwand des Spritzenkörpers (2) bildenden Kern ein Schrägschieber hervorsteht und so auf der Innenwand des Spritzenkörpers (2) den in die Wandung (3) ragenden Kanal (11) bildet, wobei der Schrägschieber nach Abschluss des Spritzgießvorganges in den Kern eingezogen wird und danach der Kern in Richtung der Öffnung (6) aus dem Hohlraum (4) des Spritzenkörpers (2) herausgezogen wird.

## Claims

1. Multi-chamber syringe (1) with a syringe body (2), of which the wall (3) encloses a hollow space (4), wherein the syringe body (2) has a syringe outlet (5) and an opening (6) for receiving a piston (7) displaceable in the hollow space (4), wherein at least one displaceable stopper (8) is arranged in the hollow space (4) and divides the hollow space (4) into a first chamber (9), for receiving a first substance, and a second chamber (10), for receiving a second substance, wherein the syringe body (2) has a channel (11) through which the first substance can flow from the first chamber (9) into the second chamber (10) when the stopper (8) is positioned in such a way that the channel (11) connects the first chamber (9) to the second chamber (10), wherein the channel (11) is formed by a material cutout in the wall (3), specifically on that side of the wall (3) facing toward the hollow space (4), **characterized in that**, starting from the syringe outlet (5), the internal diameter of the syringe body (2) widens in the direction of the opening (6) and **in that**, for the widening of the internal diameter, the inside wall of the syringe body (2) extends at an opening angle with respect to the center axis, wherein the widening of the internal diameter takes place in regions, and wherein the opening angle of the first region assigned to the channel (11) is greater than the second region assigned to the syringe outlet (5) and the third region assigned to the opening (6).

2. Multi-chamber syringe according to Claim 1, **characterized in that** the channel (11) is designed as a groove extending in the longitudinal direction of the syringe body (2).

3. Multi-chamber syringe according to Claim 2, **characterized in that** the channel (11) has a first portion (12), in which the groove base (13) of the groove extends obliquely with respect to the center axis of the syringe body (2).

4. Multi-chamber syringe according to one of Claims 1 to 3, **characterized in that** the channel (11) has a second portion (14), in which the groove base (13') extends parallel to the center axis of the syringe body (2).

5. Multi-chamber syringe according to Claim 4, **characterized in that** the first portion (12) is located closer to the opening (6), and **in that** the second portion (14) is located closer to the syringe outlet (5).

6. Multi-chamber syringe according to one of Claims 1 to 5, **characterized in that** the opening (6) of the syringe body (2) is assigned an annular grip plate (15).

7. Multi-chamber syringe according to one of Claims 1 to 6, **characterized in that** the syringe body (2) is made from a plastics injection molding material, preferably from cyclo-olefin copolymer.

8. Multi-chamber syringe according to one of Claims 1 to 7, **characterized in that** the stopper (8) and the piston (7) are made from an elastomer material, preferably from bromobutyl rubber.

9. Multi-chamber syringe according to one of Claims 1 to 8, **characterized in that** the stopper (8) and the piston (7) are provided with circumferential ribs (23).

10. Multi-chamber syringe according to one of Claims 1 to 9, **characterized in that** the syringe body (2), including the channel (11) formed in its wall, is produced by an injection molding method.

11. Method for producing a multi-chamber syringe according to one of the preceding claims, in which method the syringe body (2) is formed by means of plastics injection molding, wherein the channel (11), during the injection molding operation, is generated at the same time as the primary forming of the syringe body (2).

12. Method according to Claim 11, **characterized in that**, for the primary forming of the syringe body (2) and the simultaneous production of the channel (11), an oblique slide protrudes from the core forming the inside wall of the syringe body (2) and thus forms, on the inside wall of the syringe body (2), the channel (11) jutting into the wall (3), wherein the oblique slide is drawn into the core when the injection molding process is completed, and the core is thereafter withdrawn from the hollow space (4) of the syringe body (2) in the direction of the opening (6).

## Revendications

1. Seringue multi-chambre (1) avec un corps de seringue (2), dont la paroi (3) entoure une cavité (4), dans laquelle le corps de seringue (2) présente une sortie de seringue (5) et une ouverture (6) destinée à recevoir un piston (7) pouvant coulisser dans la cavité (4), dans laquelle au moins un bouchon coulissant (8) est disposé dans la cavité (4), lequel divise la cavité (4) en une première chambre (9) destinée à contenir une première substance et en une deuxième chambre (10) destinée à contenir une deuxième substance, dans laquelle le corps de seringue (2) présente un canal (11), à travers lequel la première substance peut s'écouler de la première chambre (9) dans la deuxième chambre (10), lorsque le bouchon (8) est positionné de telle manière que le canal (11) relie la première chambre (9) à la deuxième chambre (10), dans laquelle le canal (11) est formé par un évidement de matière dans la paroi (3) formé sur le côté de la paroi (3) tourné vers la cavité (4), **caractérisée en ce que** le diamètre intérieur du corps de seringue (2) s'élargit à partir de la sortie de seringue (5) en direction de l'ouverture (6) et **en ce que**, pour l'élargissement du diamètre intérieur, la paroi intérieure du corps de seringue (2) s'étend sous un angle d'ouverture par rapport à l'axe central, dans laquelle l'élargissement du diamètre intérieur est effectué par zones et dans laquelle l'angle d'ouverture de la première zone associée au canal (11) est plus grand que celui de la deuxième zone associée à la sortie de seringue (5) et que celui de la troisième zone associée à l'ouverture (6).

2. Seringue multi-chambre selon la revendication 1, **caractérisée en ce que** le canal (11) est réalisé sous la forme d'une rainure s'étendant dans la direction longitudinale du corps de seringue (2).

3. Seringue multi-chambre selon la revendication 2, **caractérisée en ce que** le canal (11) présente une première section (12), dans laquelle le fond de rainure (13) de la rainure s'étend en oblique par rapport à l'axe central du corps de seringue (2).

4. Seringue multi-chambre selon l'une quelconque des revendications 1 à 3, **caractérisée en ce que** le canal (11) présente une deuxième section (14), dans laquelle le fond de rainure (13') s'étend parallèlement à l'axe central du corps de seringue (2).

5. Seringue multi-chambre selon la revendication 4, **caractérisée en ce que** la première section (12) est située plus près de l'ouverture (6) et **en ce que** la deuxième section (14) est située plus près de la sortie de seringue (5).

6. Seringue multi-chambre selon l'une quelconque des revendications 1 à 5, **caractérisée en ce qu'**une plaque de prise annulaire (15) est associée à l'ouverture (6) du corps de seringue (2).

7. Seringue multi-chambre selon l'une quelconque des revendications 1 à 6, **caractérisée en ce que** le corps de seringue (2) est formé en une matière plastique moulée par injection, de préférence en copolymère de cyclo-oléfine.

8. Seringue multi-chambre selon l'une quelconque des revendications 1 à 7, **caractérisée en ce que** le bouchon (8) et le piston (7) sont formés en un matériau élastomère, de préférence en caoutchouc bromobutyle.

9. Seringue multi-chambre selon l'une quelconque des revendications 1 à 8, **caractérisée en ce que** le bouchon (8) et le piston (7) sont dotés de nervures périphériques (23).

10. Seringue multi-chambre selon l'une quelconque des revendications 1 à 9, **caractérisée en ce que** le corps de seringue (2) y compris le canal (11) formé dans sa paroi est fabriqué au cours du procédé de moulage par injection.

11. Procédé de fabrication d'une seringue multi-chambre selon l'une quelconque des revendications précédentes, dans lequel le corps de seringue (2) est formé par moulage par injection de matière plastique, dans lequel le canal (11) est produit pendant l'opération de moulage par injection en même temps que la formation initiale du corps de seringue (2).

12. Procédé selon la revendication 11, **caractérisé en ce que**, pour la formation initiale du corps de seringue (2) et la fabrication simultanée du canal (11), un curseur oblique est saillant sur le noyau formant la paroi intérieure du corps de seringue (2) et forme ainsi sur la paroi intérieure du corps de seringue (2) le canal (11) pénétrant dans la paroi (3), dans lequel le curseur oblique est rétracté dans le noyau après la fin de l'opération de moulage par injection et le noyau est ensuite retiré hors de la cavité (4) du corps de seringue (2) en direction de l'ouverture (6).
